# EUROPEAN PATENT APPLICATION

(11) **EP 2 311 401 A1**
(43) Date of publication of application: **20.04.2011**
(21) Application number: 10187815.5
(22) Date of filing: 15.10.2010
(51) Int. Cl.: A61B 19/00

(54) **Non-invasive dental based fiducial array**

(30) Priority: 15.10.2009 US 251967 P
(71) Applicant: Hybex Holdings, Inc., Montreal QC H1E 6P1 (CA)
(72) Inventor: Hynes, Brian, Montreal Québec H1E 6P1 (CA); Comeau, Roch M., Montreal Québec H3J 2P2 (CA); Frey, Stephen, Montreal Québec H4A 2W5 (CA)
(74) Representative: Dempster, Benjamin John Naftel

(57) **Abstract**

The invention relates generally to apparatus and devices for neuronavigation. A non-invasive dental based fiducial array (20) is disclosed. The fiducial array has a base support member (22), one or more removable and repositionable marker bases (86) supported by the base support member and a stopper (50) spaced from and supported by the base support member that engages a part of the head of a patient or an animal and cooperates with the base support member to immobilize the fiducial array relative to a location of the head, such as maxillary teeth. An anchoring site (70) is formed on the base support member to accept a custom made dental impression to precisely position the base support member relative to the location of the head.

## Description

### Field of Invention

The invention relates generally to apparatus and devices for neuronavigation. More particularly, a non-invasive dental based fiducial array is disclosed.

### Background of Invention

It is known to use fiducial arrays to provide fiducial points for neuronavigation. Markers attached to a fiducial array provide the required reference points in an image scan. While fiducial arrays may be attached to a head by employing anchoring holes drilled in the skull, non-invasive fiducial arrays are often preferred.

For example, there are known dental based non-invasive fiducial array such as those described in United States Patent Nos. 6,096,048 and 6,223,067 and United States Patent Application Publication No. US2004/0015176. However, often, it is desirable that a dental based fiducial array can be removed from a patient's mouth (or an animal's mouth) and reinstalled later very accurately to the original position in the mouth. The accuracy is crucial to the precision of image scan and for registration of the imaged anatomy to the scanned images. Further, structural members of a fiducial array may not always be suitably positioned for patients of all ages. Similarly, different geometries of animals of different species may also cause interferences with part of the geometries or require re-positioning of fiducial markers to different locations to closely follow the location of brain regions.

The forgoing creates challenges and constraints for a fiducial array for providing easily locatable fiducial points for neuronavigation and other similar procedures. There is therefore a need for a fiducial array as compared to the existing art. It is an object of the present invention to mitigate or obviate at least one of the above mentioned disadvantages.

### Summary of Invention

The invention relates generally to apparatus and devices for neuronavigation. More particularly, a non-invasive dental based fiducial array is disclosed. One aspect of the present invention involves a fiducial array that includes a base support member, one or more removable and repositionable hubs supported by the base support member and a stopper spaced from and supported by the base support member that engages a part of the head of a patient or an animal and cooperates with the base support member to immobilize the fiducial array relative to maxillary teeth. An anchoring site is formed on the base support member to accept a custom made dental impression to precisely position the base support member relative to the maxillary teeth. The hubs have removable marker base attached thereto for supporting fiducial markers.

In an aspect of the invention, a non-invasive, dental-based fiducial array is provided. The fiducial array includes a base support member, a support arm, and one or more marker bases removably and re-positionably mounted to the support arm, each of said one or more marker bases being adapted for mounting a fiducial marker. The base support member is shaped to be received in oral cavity of a person or an animal and includes an anchoring site for accepting a customized dental impression. The support arm is pivotally secured to the base support member.

One feature of the aspect of the invention provides a fiducial array that further includes a stopper spaced from and re-positionably mounted to the base support member. The stopper is sized to engage the head of the person or the animal and cooperating with the base support member to immobilize the base support member relative to the head. As a further feature, the fiducial array also includes a mounting post slidably coupled to the base support member and a mounting block releasably secured to the mounting post, the stopper being re-positionably secured to the mounting block.

According to another feature of the aspect of the invention, the fiducial array further includes a removable hub mounted on the support arm, the one or more marker bases being mounted to the support arm through the removable hub.

According to yet another feature of the aspect of the invention, the fiducial array further includes a connection member that pivotally connects the support arm to the base support member. According to a further feature, the connection member includes two pivot connections and the two pivot connections have non-parallel pivot axes.

In other aspects the invention provides various combinations and subsets of the aspects described above.

### Brief Description of Drawings

For the purposes of description, but not of limitation, the foregoing and other aspects of the invention are explained in greater detail with reference to the accompanying drawings, in which:

Figure **1** is a perspective view showing a dental based fiducial array;

Figure **2** is a cross-sectional view showing a connection link that pivotally connects support arm of the fiducial array of Figure 1 to its base support member;

Figure **3** illustrates in a perspective view a base support member of the fiducial array shown in Figure **1****;**

Figure **4** shows in a perspective view an upright post;

Figure **5** shows in a perspective view a support arm;

Figure **6** shows in a perspective view an alternative support arm;

Figure **7** shows in a perspective view a removable hub (with some spokes and fiducial marker discs removed for better illustration) pivotally supported on a base plate of the fiducial array shown in Figure **1****;**

Figure **8** is a perspective view showing a dental based fiducial array that includes a stopper;

Figure **9** is a perspective view a stopper of the fiducial array of Figure **8****;**

Figure **10** shows in a cross-sectional view a stopper that includes a slide mount secured to a mounting post which is in turn secured to a base plate;

Figure **11** shows in a perspective view another construction of a dental-based fiducial array; and

Figure **12** shows in an exploded view the dental-based fiducial array of Figure **11****.**

### Detailed Description of Embodiments

The description which follows and the embodiments described therein are provided by way of illustration of an example, or examples, of particular embodiments of the principles of the present invention. These examples are provided for the purposes of explanation, and not limitation, of those principles and of the invention. In the description which follows, like parts are marked throughout the specification and the drawings with the same respective reference numerals.

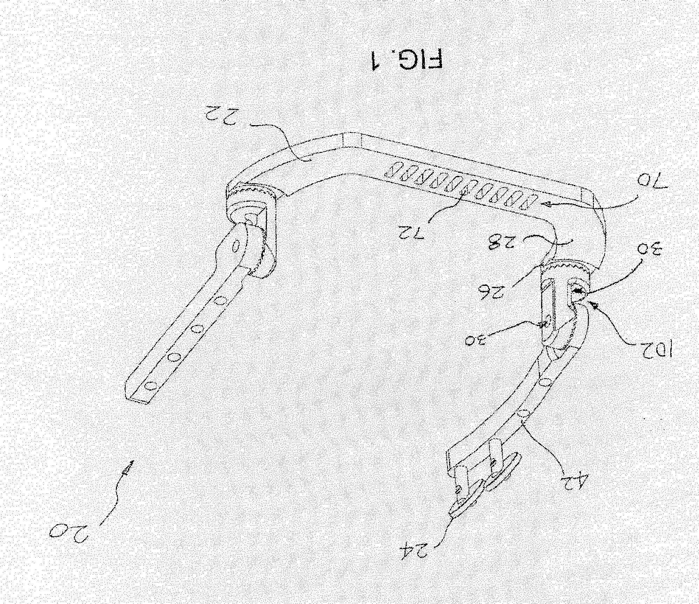

Referring to Figure **1****,** there is shown an embodiment of a dental based fiducial array, generally indicated by reference number **20.** Fiducial array **20** has a base support member such as a base plate **22.** Base plate **22** may have a general U-shape, or some other shapes to be received in oral cavity of a person or an animal so that the person or animal may bite part of the base plate to immobilize the base plate with respect to a location of head, such as upper or lower jaw, or maxillary teeth or upper palate, while some other part of the base plate can be exposed for supporting other components of the fiducial array, without interfering with the anatomy of the oral cavity or the head. Base plate can be sized to fit human and/or animal mouth sizes. It will be appreciated, however, that the base plate may also take other shapes, such as an arc, a plate having an inner curved edge to accommodate the jaw anatomy, among others. As will be described in detail later, base plate provides an anchor point for precise positioning of the fiducial array relative to a location of the head, such as maxillary teeth of a person or animal. The exposed portion of the base plate can also be used for positioning the person or the animal's head relative to other medical instrument where needed.

The base plate **22** has an anchoring site **70** for receiving a custom made dental impression. The anchoring site **70** has a plurality of slots **72** (or holes or other anchoring arrangements) formed in the base plate **22.** A dental impression of maxillary teeth (and if desired, upper palate) of a patient or an animal is formed from a thermo-moldable dental plastic and includes a plurality of projections to be received in the plurality of slots **72.** Conveniently, the thermo-moldable dental plastic may be placed on the base plate **22** with the maxillary teeth pressed against the base plate to make the dental impression. The pressure applied tends to create a plurality of projections that extend into and match the shape and location of the plurality of slots, thus providing desirable fitting between the dental impression and the base plate.

One or more removable marker support, such as marker discs **24** are removably and pivotally joined to and supported by the base plate **22.** As will be described in detail later, each marker disc **24** is adapted for mounting a marker, which provides locatable fiducial points for neuronavigation. A support arm **42** may be provided for mounting the marker discs. A connection member, such as connection link **102** that includes a pivot connection or connections **30,** pivotally joins the support arm **42** to the base plate. Pivot connections allow the marker discs to be repositioned relative to the base plate, when required. Figure **1** shows a U-shaped base plate. Connection link is mounted to the base plate **22** at the distal ends **26** of the legs **28** of the U. This allows the markers to be placed as close to the brain region as possible, often of interest to a neurosurgeon.

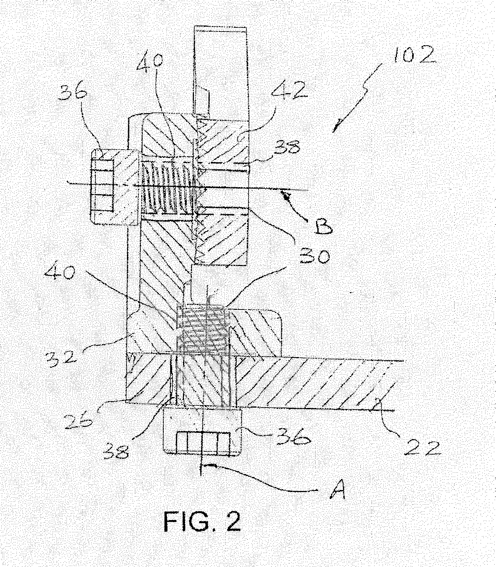

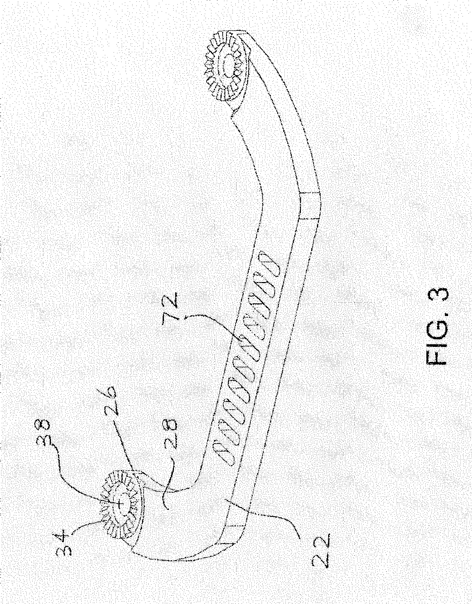

Connection link **102** shown in Figure **1** and Figure **2** has two pivot connections **30,** each pivot connection having a pivot axis A, B, and pivot axes A, B being non-parallel. Referring to Figures **1** to **5****,** an upright post **32** is pivotally joined to the base plate adjacent its distal end **26.** The upright post **32** has a toothed connection surface **34** to mate with a corresponding toothed connection surface **34** formed on the base plate **22** (Figure **3**). A securing screw **36** or such other appropriate fasteners can be used to pass through a throughhole **38** formed in the base plate and threaded into a threaded hole **40** formed in the upright post to releasably secure the upright post to the base plate **22.** As can be more clearly seen in Figure **2****,** upright post **32** is pivotable about rotational axis A of the securing screw **36.** Thus, connection between the upright post to base plate provided by the securing screw **36** allows the upright post **32** to pivot about the pivot axis A and provides the first pivot connection **30.** Tightening the securing screw **36** urges the toothed connection surfaces **34** toward each other until stopped and thereby fixes the orientation of the upright post **32** about its pivot axis. The toothed connection surfaces **34** define a series of pre-selected angular positions and facilitate precise angular positioning of the upright post.

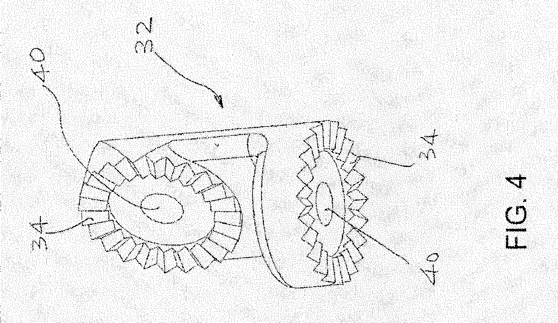

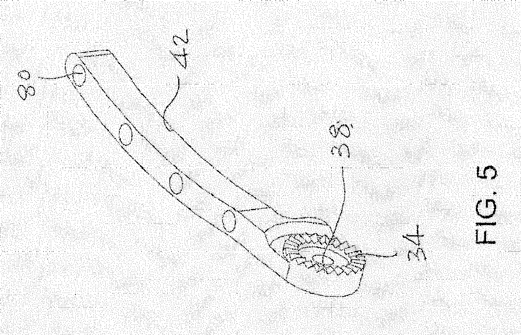

A support arm **42** is pivotally joined to the upright post **32** at a location spaced from the base plate. A securing screw **36** is used to provide a second pivot axis B and to secure the support arm **42** to the upright post **32.** Similarly, matching toothed connection surfaces **34** may be formed on support arm **42** and upright post **32,** as shown in Figures **4** and **5****,** for precise angular positioning of support arm **42** relative to upright post **32.** Throughhole **38** formed in support arm **42** and threaded hole **40** in upright post **32** allow a securing screw **36** to pass through and secure the support arm to the upright post. The connection between support arm **42** and upright post **32** provides the second pivot connection **30,** i.e., a pivot connection pivotable about pivot axis B. Of course, a threaded hole may also be formed in support arm **42** and a throughhole formed in upright post **32,** or throughholes be formed in both support arm **42** and upright post **32,** in which case a nut and a bolt will be required.

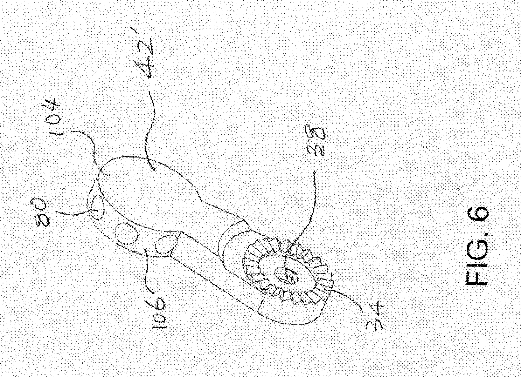

Support arm **42** may be adapted for direct attachment of fiducial marker discs **86.** Figure **5** illustrates a support arm **42** that has a series of spoke holes **80** formed along one of its sides, each of which is suitably sized for accepting a spoke **84** that supports a fiducial disc **86** thereon. Support arm may be straight or suitably curved as seen in Figure **1****.** One support arm **42** shown in Figure **1** is suitably curved so that the markers can be positioned as close to the head of a patient or animal as possible. Figure **6** illustrates another example of a support arm for direct attachment of fiducial marker discs. Support arm **42'** shown in Figure **6** has a round terminal end **104.** A series of spoke holes **80** are formed on the periphery side **106** of the terminal end **104** for accepting spokes **84** to mount fiducial marker discs **86** thereon.

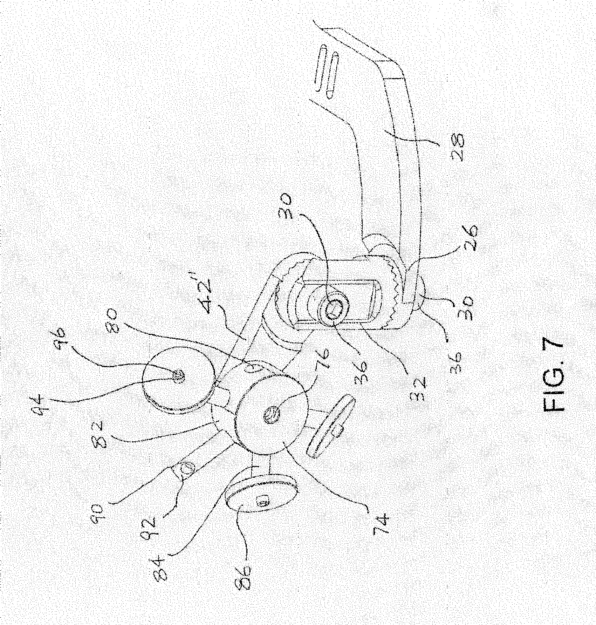

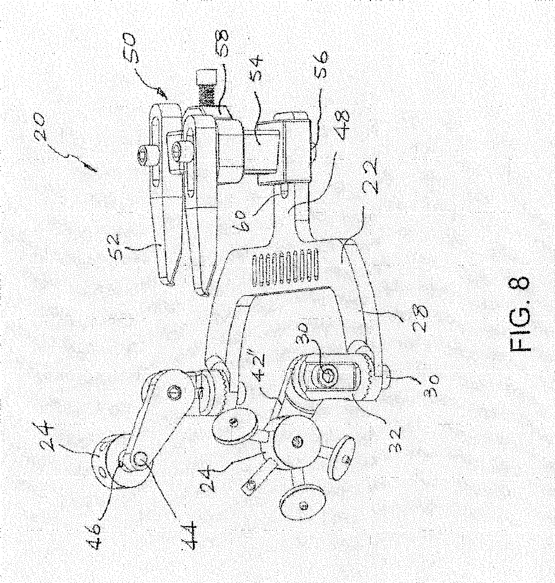

As yet another alternative, instead of forming a round terminal end at the distal end of support arm, one or more removable hubs **24** may be provided. As the pivot axes A, B of the two pivot connections **30** are in different planes and oriented differently, removable hub **24** attached to support arm also has at least two degrees of freedom. Referring to Figures **7** and **8****,** a removable hub **24** can be attached to support arm **42"** by way of a short arm **44.** A screw **46** or pin secures the removable hub **24** to the short arm **44.** Each removable hub **24** has a generally cylindrical body **74,** with a central hole **76** for receiving a short arm **44** of support arm **42"** and a plurality of spoke holes **80** formed on and angularly spaced along the side surface **82** of the cylinder. A spoke **84** is received in a spoke hole **80** and supports a fiducial marker base **86.** Spoke holes **80** may be formed along a generally radial direction and transverse to the longitudinal axis of the cylinder. They may also be at an angle different from **90°** with the longitudinal axis of the cylinder. For example, the angle may be between **60°** and **90°.**

The orientation of the removable hub **24** relative to the support arm **42"** may be adjusted by turning it around short arm **44** prior to tightening the securing screw **36.** Alternatively, short arm **44** may have a non-circular cross-section, with the hub having a hole of matching cross-sectional shape for receiving the short arm. For example, the cross section may be a hexagon, or some other polygon. The non-cylindrical cross-section allows accurate orientation of the hub relative to the support arm and any subsequent repositioning. With a cross-sectional shape such as hexagon that has rotational symmetry, stepped adjustment of relative orientation of the hub will also be possible. Of course, other means may be provided for accurate repositioning, such as markings on the hub and the short arm or the support arm.

As noted, a fiducial marker base **86** is supported at the top end **90** of a spoke **84.** Fiducial marker base **86** and spoke **84** may be individually formed and then joined together, or integrally formed as one piece. Fiducial marker base shown in Figure **1** and Figure **7** has the shape of a disc, though other shapes are also possible. The fiducial marker disc **86** is secured to the spoke. The top end of the spoke has a supporting surface **92.** The orientation of supporting surface **92,** i.e., the normal of the surface, may not be aligned with the longitudinal axis of the spoke **84.** Instead, the supporting surface may be inclined, with the normal and the longitudinal axis of the spoke **84** forming a non-zero inclination angle. With markers positioned on supporting surfaces at such an inclination angle, the markers will not be co-planar, which may be desirable for certain applications. The inclination angle may be conveniently selected to be **30°,** though it may be an angle up to 45°, or zero (i.e., not inclined).

A fiducial marker disc **86** is adapted for accepting fiducial markers (not shown). At the center of the fiducial marker disc **86** there may be provided a small nipple **94,** for fitting a self-adhesive multi-modality marker. A receiving hole may be formed in spoke **84** as shown in Figure **6** for receiving the small nipple. The nipple may have a nipple hole **96** (about 1.5 mm), into which the distal tip of a neuronavigation pointer (not shown) can be touched to co-register the location of the supported fiducial marker to scan imaging data.

Conveniently, a stopper **50** is provided for immobilizing the base plate relative to a location of the head, such as upper or lower jaw. Referring to Figure **8****,** a base plate may be configured for supporting the stopper **50,** for example, by integrally forming an extended leg **48** at the bottom segment of U for mounting the stopper thereon. A stopper may be in the form of an eye bar **52** or a set of eye bars **52.** Stopper **50** engages a region of the head of the patient or the animal. Eye bar **52** is spaced from base plate. Its height relative to the base plate **22** is adjustable so that appropriate pressure can be applied by the cooperating stopper and base plate **22** to increase the position retaining power of the fiducial array. As will be appreciated, with an extended leg **48** integrally formed with base plate **22,** the extended leg **48** may also be used for supporting other instruments on the base plate, or for attaching the base plate to a support structure or surface to immobilize head during imaging or surgery procedure, where desirable or required.

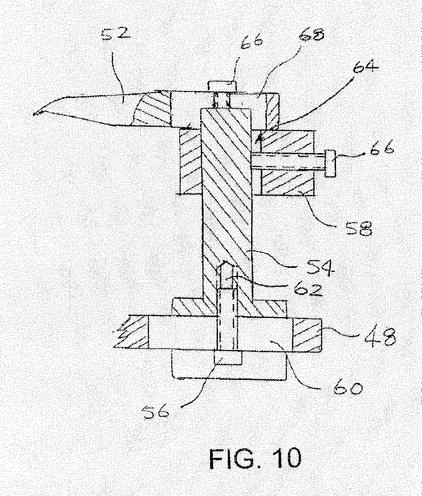

Referring to Figures **8****,** **9** and **10****,** a mounting post **54** is secured to the extended leg **48** by a securing bolt **56.** Eye bar **52** (or set of eye bars) is supported by a slide mount **58** that is slidably attached to the mounting post for adjusting height of the eye bar **52** relative to the base plate **22.** A slot **60** is formed in the extended leg **48.** A bolt hole **62** is formed in the mounting post **54.** A securing bolt **56** passes through the slot **60** and is threaded into the bolt hole **62** of the mounting post to secure the mounting post to the base plate. The slot **60** allows the mounting post **54** to be positioned at any location along the slot as may be desired. Thus, both height of eye bar **52** and distance of eye bar **52** along extended leg **48** can be adjusted and eye bar **52** can be re-positioned by adjusting its height and distance as desired or needed.

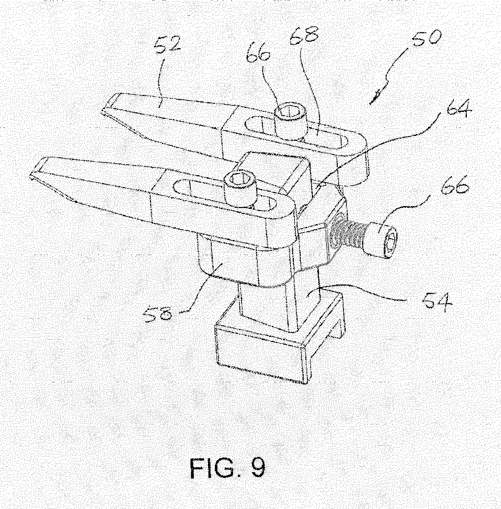

The transverse cross section of mounting post **54** may have any suitable shape. Figures **8** and **9** show an example that has a trapezoidal shape. The slide mount **58** has a corresponding trapezoidal through hole **64** for the trapezoidal shaped mounting post **54** to pass therethrough. A tightening screw **66,** or other suitable engagement means, is provided for pulling the mounting post towards the slide mount, along a direction from the longer side to the shorter side of the trapezoidal. Eye bar **52** has an eye bar slot **68** for a tightening screw **66** to pass through and to secure the eye bar to the slide mount **58.** The eye bar slot allows the eye bar to be positioned differently as the eye bar slides relative to the slide mount **58.**

Of course, although a stopper in the form of a height-adjustable eye bar is described, the stopper may take any other suitable form. For example, a strap system (or even much simpler surgical tape) can be used to replace the eye bar for immobilizing the base plate relative to the maxillary teeth (or upper palette).

The device described herein allows it to be applied to patients of different ages and also to animals of different species. To use the device for a patient (or an animal), a suitably sized base plate **22** is selected and placed in the mouth of the patient or animal. A suitable mass of thermo-moldable dental plastic, already appropriately heated, can be placed on the base plate for making a customized dental impression. Once solidified, the dental impression tends to be securely attached to the base plate, which will provide accurate anchor points when the fiducial array is reinstalled in the mouth of the patient or animal, from whom the dental impression is made.

One or more marker discs are installed for mounting fiducial markers. Position of the marker discs, or fiducial markers, can be adjusted by rotating (i.e., pivoting) the upright post 32 and the support arm **42.** The pivot connections **30** provided by the upright post and the support arm allow the marker discs, or removable hubs if used for supporting marker discs, to be placed as close to patient's head (or the animal's head) as possible and close to the brain area which is of interest to a neurosurgeon. Once the marker discs or the removal hubs are suitably positioned, the securing screws **36** can be tightened. It is possible that one or more of the spokes **84** or fiducial marker discs **86** may interfere with some parts of the patient's (or the animal's) anatomy. The interfering fiducial marker disc or discs can be removed or the interfering spoke(s) be removed to avoid interference. The position of the removable hub or hubs and the fiducial marker discs may be recorded or left unchanged for future use.

When the fiducial array needs to be reinstalled, the custom made dental impression is matched with the dental structures of the patient or animal, such as formation of maxillary teeth or upper palate, to ensure accurate positioning of the base plate **22** and the fiducial marker discs **86** carried by the base plate. The positions of fiducial marker discs may be further fine tuned should there be any relocation of these marker discs since last image scan. The height and position of stoppers **50** can be suitably adjusted to immobilize the fiducial array **20** during the surgical, imaging or other medical procedures.

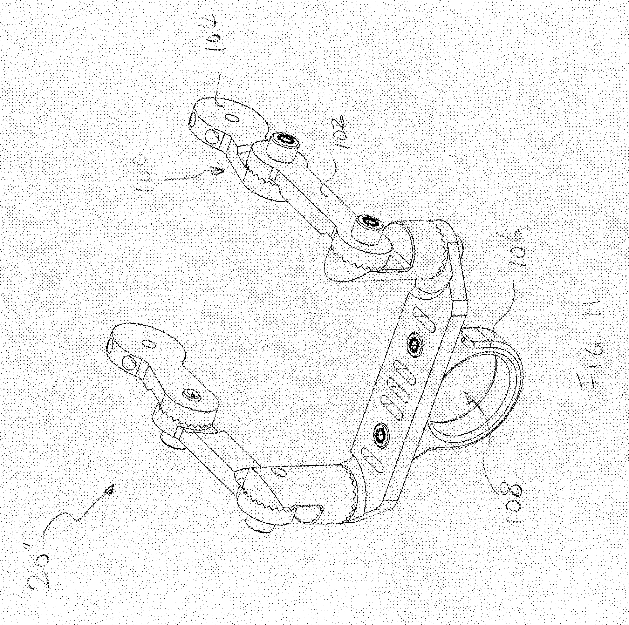

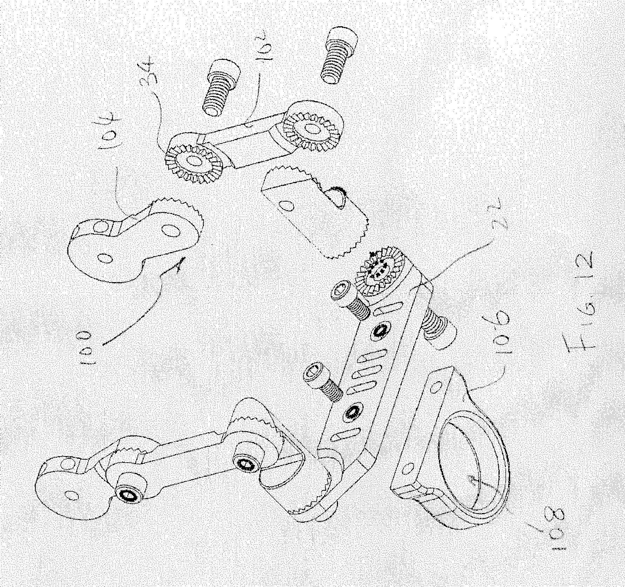

Figures **11** and **12** show in a perspective view and an exploded view another construction of a dental-based fiducial array **20".** Fiducial array **20"** is similar in construction to fiducial array **20** shown in Figure 1. Unlike the single-piece construction of support arm **42** of fiducial array **20,** support arm **100** has a multi-section construction. As shown in Figures **11** and **12****,** support arm **100** has a first section **102** and a second section **108,** pivotally joined to each other. Toothed connection surfaces **34** can be provided at each pivot connections to define a series of pre-selected angular positions and facilitate angular positioning of neighboring sections. Although only two sections are shown in Figures **11** and **12****,** it will be understood that more sections may be used where appropriate and feasible. The distal section, or the second section **104** in this case, is shown to be the same as the short support arm **42** that has a round terminal end for mounting fiducial marker discs as described in connection with fiducial array **20** and illustrated in Figure **6****.** Support arm **100** may also include holes **80,** similar to that shown in Figure **1****,** or hubs **24** similar to that shown in Figure **7****,** for mounting fiducial marker discs.

Fiducial array **20"** also includes an attachment **102** which has a throughhole **104** defined therein. The attachment is shown to be secured to base plate **22** by threaded nuts, though it may also be secured using any other suitable fasteners. The attachment may also be integrally formed with base plate **22.** When the fiducial array is placed in the mouth of a patient or animal, the attachment provides support so that the mouth may remain open for longer duration without causing excessive fatigue. The throughhole **108** can be used to accommodate endotrached tube ("ET tube") or other tubing during surgery. Tubings that pass through the throughhole **108** thus are protected and the mouth of the patient or the animal would not be closed so as to squeeze the ET tube or other catheters or probes in the mouth. The attachment **102** can also be used together with a strap to keep the fiducial array **20"** in position in the mouth of an animal. For example, the fiducial array **20"** may be placed in an animal's mouth so that the lower jaw and the maxillary teeth can close on the attachment **104** and the dental impression secured to base plate **22.** A strap may be provided to help keep the mouth closed.

Various embodiments of the invention have now been described in detail. Those skilled in the art will appreciate that numerous modifications, adaptations and variations may be made to the embodiments without departing from the scope of the invention. Since changes in and or additions to the above-described best mode may be made without departing from the scope of the invention, the invention is not to be limited to those details but only by the appended claims.

## Claims

1. A dental-based fiducial array for providing re-positionable fiducial points required by neuronavigation, the fiducial array comprising:
a base support member, the base support member being shaped to be received in oral cavity of a person or an animal and including an anchoring site for accepting a customized dental impression,
a support arm, said support arm being pivotally joined to the base support member, and one or more marker bases removably and re-positionably mounted to the support arm, each of said one or more marker bases being adapted for mounting a fiducial marker.

2. The fiducial array of claim 1, further comprising a stopper spaced from and re-positionably mounted to the base support member, said stopper being sized to engage the head of the person or the animal and cooperating with the base support member to immobilize the base support member relative to the head.

3. The fiducial array of claim 2, further comprising a mounting post slidably coupled to the base support member and a mounting block releasably secured to the mounting post, the stopper being re-positionably secured to the mounting block.

4. The fiducial array of claim 1, wherein said base support member has a throughhole defined therein for tubings to pass therethrough.

5. The fiducial array of claim 1, wherein said base support member includes an attachment having said throughhole defined therein.

6. The fiducial array of claim 1, further comprising a removable hub attached to the support arm for mounting the one or more marker bases, the one or more marker bases being mounted to the support arm through the removable hub.

7. The fiducial array of claim 6, wherein said removable hub has a plurality of holes formed thereon for accepting the one or more marker bases, each of said one or more marker bases having a post to be received in one of said plurality of holes.

8. The fiducial array of claim 1, wherein said support arm has a plurality of holes formed thereon for accepting the one or more marker bases, each of said one or more marker bases having a post to be received in one of said plurality of holes.

9. The fiducial array of claim 8, wherein said post defines a longitudinal axis, said each marker base having a support surface for mounting said fiducial marker, normal direction of said support surface and said longitudinal axis forming a non-zero inclination angle.

10. The fiducial array of claim 9, wherein the non-zero inclination angle is less than 45°.

11. The fiducial array of claim 1, wherein said support arm comprises a plurality of sections pivotally joined to each other.

12. The fiducial array of claim 11, wherein said pivotally joined sections are configured to be releasably secured to one of a plurality of pre-selected angular positions.

13. The fiducial array of claim12, wherein each of said pivot connections has matching toothed connection surface formed on, said toothed connection surfaces defining said plurality of pre-selected angular positions.

14. The fiducial array of claim 1, further comprising a connection member, said connection member pivotally joining said support arm to the base support member.

15. The fiducial array of claim 14, wherein said connection member includes a first pivot connection and a second pivot connection, said first and second pivot connections having non-parallel pivot axes, said first connection connecting said connection member to the base support member, said second connection connecting said connection member to said support arm.

16. The fiducial array of claim 15, said first and second pivot connections each having a plurality of pre-selected angular positions and each of said pivot connections being configured to be releasably secured to one of said plurality of angular positions.

17. The fiducial array of claim 16, wherein each of said first and second pivot connections has toothed connection surface formed on said connection member, said toothed connection surface defining said plurality of pre-selected angular positions.

18. The fiducial array of claim 1, wherein said anchoring site includes a plurality of slots for accepting and securing thereto said dental impression.
